Europäisches Patentamt

European Patent Office

Office européen des brevets

(19)

(11) Publication number: 0 393 961 A2

# EUROPEAN PATENT APPLICATION

(12)

(21) Application number: 90304050.9

(22) Date of filing: 12.04.90

(51) Int. Cl.5: G01N 33/543, G01N 33/52, //G01N33/53

(30) Priority: 19.04.89 US 340245

(43) Date of publication of application:
24.10.90 Bulletin 90/43

(84) Designated Contracting States:
DE FR IT

(71) Applicant: PALL CORPORATION
30 Sea Cliff Avenue
Glen Cove New York 11542(US)

(72) Inventor: Harwood, Colin F.
17 Southland Drive
Glen Cove, New York 11542(US)
Inventor: Pascale, Frank R.
12 Francis Court
Glen Cove, New York 11542(US)
Inventor: Matkovich, Vlado I.
11 Old Estate Road
Glenn Cove, New York 11542(US)

(74) Representative: Knott, Stephen Gilbert et al
MATHISEN, MACARA & CO. The Coach
House 6-8 Swakeleys Road
Ickenham Uxbridge Middlesex UB10 8BZ(GB)

(54) Porous, absorbent, multiple element diagnostic device.

(57) A diagnostic device is provided comprising a receptacle having an open bottom, and a multiple element diagnostic structure secured to the receptacle and disposed across the open bottom, the multiple element diagnostic structure comprising a porous, liquophilic membrane, and a liquid absorbent, wettable, porous element having a low non-specific affinity for amide group-containing substances, as measured by the Immersion Protein Binding Assay located below and in contact with said porous liquophilic membrane. The multiple element diagnostic structure may also include a supplemental liquophilic, liquid absorbent structure.

EP 0 393 961 A2

# POROUS, ABSORBENT, MULTIPLE ELEMENT DIAGNOSTIC DEVICE

The present invention is directed to a diagnostic device capable of producing uniform and reproducible test results. More particularly, the present invention is directed to a diagnostic device which includes a porous multiple element structure capable of providing which assures rapid and uniform unassisted removal of liquid from a test zone.

In recent years, medical research has led to a proliferation of diagnostic tests and techniques as well as the apparatus and devices employed to carry out such tests. ELISA assays, RNA and DNA hybridization assays, and microbiological assays are but a few of the types of diagnostic test methods which have been developed recently. In addition to providing a diagnostic test procedure for a particular bacteria, virus, antigen, etc., greater sensitivity, accuracy (fewer false positives), rapidity, and simplification have also been objectives sought in developing test procedures and devices. Such devices have frequently taken the form of a reservoir or receptacle in the form of a tube or well having a porous structure, typically a membrane, secured to an open bottom portion of the reservoir. This porous or microporous membrane generally functions in one of two ways: (1) it acts as a reaction layer in which a test reagent immobilized on or within the microporous membrane provides an indicia of the presence or absence of a specific analyte, or (2) it may function to retain on its upper surface small test particles, typically plastic particles, such as polystyrene spheres or beads which are provided with a test reagent on their surfaces for the detection of an analyte.

To provide maximum sensitivity, whether using a membrane in which the test reagent is immobilized at its surface (type (1)) or reagent-containing plastic beads are retained thereon (type (2)), maximum surface area of the membrane is sought. Thus, with devices of type (1), the higher the surface area the more test reagent may be immobilized on the surface and the higher the sensitivity. This is achieved by employing a microporous membrane. With devices of type (2), surface area varies inversely with particle size of the beads employed. As smaller particles are employed to achieve high surface area, the pores of the membrane are, accordingly, smaller, or at least the surface adjacent the beads has smaller pores, to avoid penetration of the pores by the beads. Thus, the membrane is also microporous or the gross surface adjacent the beads is microporous.

Although in some instances it is necessary to allow either a suspected analyte-containing liquid or a reagent-containing liquid to remain in the well above the surface of the membrane to assure adequate incubation or development of a reaction product which indicates a positive test, it is also necessary in many instances to remove the liquid from the reservoir at a controlled and, preferably, rapid rate. Liquid removal may be achieved in a number of ways. Each method, however, requires some driving or motive force which induces or forces liquid through the membrane. Typically these methods involve principles of gravity flow, differential pressure, or wicking. The first method, employing gravity flow, may be effective if the height of the reservoir or tube is sufficiently great to accommodate a tall column of fluid and/or the pores of the membrane are sufficiently large. However, with the above-discussed requirements of surface area and the use of a microporous membrane, along with the practical considerations of using a tube or well with a relatively short height in order to facilitate transfer of liquids and permit ease of viewing the membrane or beads positioned on the upper surface of the membrane, the height of the reservoir is typically too short to permit unassisted gravity flow of liquid through a microporous membrane at a reasonable rate.

The use of either elevated pressure or, more commonly, vacuum equipment to effect a differential pressure across the membrane in a diagnostic device in which such membrane is located at the bottom of a liquid reservoir provides an effective method of removing liquid from the reservoir. However, this technique requires somewhat elaborate equipment such as a pump to create an elevated pressure or a "source" of vacuum and a manifold, such as a vacuum manifold, to hold the reservoir containing devices, such as tubes or a well-containing plate, and to maintain the desired pressure. Although many laboratories may have such equipment, there are some which do not and the requirement of such equipment forecloses the ability to perform simple diagnostic tests in the home or small laboratory. In addition, although not demanding a high skill level, the use of such equipment does require the user to have some experience with this type of equipment.

A third method of creating a motive force through the membrane involves a two element wicking structure. The first of the two elements is the microporous membrane which serves as the reaction layer or retention layer for the test reagent-containing plastic beads. The second element is an absorbent member which is formed from a porous liquid absorbent material. The liquid absorbent member is placed below and in contact with the lower surface of the microporous membrane, that is, the surface opposite the test surface or that surface which supports the plastic test reagent coated beads. A liquid sample placed in the reservoir contacts the upper surface of the membrane and begins to pass through and saturate the membrane. When the liquid reaches the lower surface of the membrane, it contacts the absorbent member and is rapidly drawn through the membrane due to a wicking or capillary action of the liquid absorbent member.

Although such two element diagnostic structures are effective in eliminating liquid from a test sample placed in a reservoir above the two element structure and are easy to use even by the layman, such devices formed from conventional materials suffer from certain drawbacks. Reproducible and uniform flow through the test or retaining microporous membrane is required if test results are to be consistent and reproducible. A rapid flow rate through the membrane is required if the typical test is to be practical application. If irregular flow patterns develop in the membrane or if excessive flow times occur, false positive or negative results may be indicated. To achieve optimal flow characteristics, effective and uniform absorption across the lower gross surface is a requirement. However, there exists a critical problem with such two element structures employing conventional materials which can impede effective absorption. This results from air or gas bubbles being trapped between the membrane layer and the pad of absorbent material, frequently in an irregular manner across the lower gross surface of the membrane. The formation of such air bubbles results from the inherent nature of the materials used and the structure of such a diagnostic device. In use, the liquid sample must first wet the membrane and thereafter flow through the depth of the membrane to contact the absorbent pad located therebelow and in contact with the membrane. The variations in intimacy of contact between the membrane and the absorbent pad and, perhaps, differences in the time at which the liquid front emerges from the membrane leads to liquid contacting the absorbent pad in some areas before others. As a result, air bubbles are often trapped between the membrane and pad during the initial wetting. Once trapped, these air bubbles cannot escape, and they effectively block liquid flow for the remainder of the test in the areas of the membrane superposed above them. Since such two element diagnostic devices rely solely on the vertical component of capillary action to draw the liquid from a sample through the membrane layer, any air trapped between the membrane and the absorbent pad can seriously impede, if not completely block, the flow of liquid through certain areas of the membrane layer. These blocked areas exhibit different flow characteristics than the rest of the membrane and may result in false positive, false negative, or indeterminant readings in a test.

In an attempt to overcome this problem and improve contact and communication between the membrane and the absorbent pad, modifications of such two element diagnostic structures have been tried. Thus, a three element or three layer device has been employed in which inter-layers of a fluffy fibrous material is placed intermediate the membrane and the absorbent pad, the purpose of the inter-layers being to fill and bridge any voids between the membrane and the absorbent pad. However, like the two element structures described above, intimacy between all layers of a three element structure prepared from conventional materials appears to be a prerequisite for proper function and may be compromised by slight differences in assembly from one device to the next. Insufficient compression of the layers may also result in poor communication between layers, even with the additional fluffy inter-layers interposed between the membrane and the absorbent pad. Furthermore, excessive compression can cause the membrane to bulge away from the lower structures, also resulting in similar degraded inter-layer communication.

A different problem also occurs with the membranes commonly employed with diagnostic test devices in current use. Many of the liquid samples tested with such diagnostic devices contain proteinaceous material. Many of the tests themselves depend upon reaction of a test reagent with a specific analyte which is typically a protein. The microporous membranes frequently used in such diagnostic devices have an affinity for proteinaceous materials and tend to absorb or bind such materials present in the sample. The absorption is both specific and nonspecific. The binding of nonspecific protein can lead to false positive results, thus making the test unreliable. In addition, the conventional materials used both as the membrane material as well as the absorbent pad or the intermediate absorbent layer tend to be translucent once wetted. In many situations, protein-containing sample liquid, test reagents (some of which are colored), and wash solutions collect in or below the liquid absorbent pad. Because of the colored nature of the reagents and/or reactions which occur in the liquid phase and the translucent character of the membrane, and perhaps the absorbent pad and/or the intermediate absorbent layer, a colored background may be observed which could give misleading results.

To avoid such absorption of nonspecific protein, a deactivating or blocking treatment is performed which renders the membrane inert or passive toward extraneous or nonspecific proteins in the liquid which is assayed. Although somewhat effective in minimizing nonspecific absorption of protein, the blocking treatment does have certain shortcomings. First, depending on the type of membrane being treated, technical difficulties may deter an even and thorough saturation of the membrane with blocking solution. In addition, substances used in the blocking treatment and in treating the membrane to minimize bacterial growth during storage are sometimes toxic and thereby present a health hazard to those people who are frequently exposed to the materials used in the blocking treatment, particularly those who are engaged in manufacturing operations.

The present invention provides a diagnostic device comprising: (a) a receptacle having an open bottom; and (b) a multiple element diagnostic structure secured to said receptacle and disposed across the open bottom said multiple element diagnostic structure comprising: (i) a porous, liquophilic membrane, and (ii) a liquid absorbent, wettable, porous element having a low non-specific affinity for amide group-containing substances, as measured by the Immersion Protein Binding Assay located below and in contact with said porous, liquophilic membrane. The liquid absorbent, wettable, porous element may comprises a non-woven element of thermoplastic, liquophilic fibers having a low affinity for amide group-containing substances, and the amide group-containing substances may comprise proteinaceous substances. The fibers further may be hydrophilic.

The present invention also provides for a multiple element diagnostic structure comprising: (a) a porous, liquophilic membrane, and (b) a liquid absorbent, wettable, porous element having a low non-specific affinity for amide group-containing substances, as measured by the Immersion Protein Binding Assay located below and in contact with said porous, liquophilic membrane. Furthermore, the multiple element diagnostic structure may include a supplemental, liquophilic, liquid absorbent element located below and in contact with said liquid absorbent, wettable, porous element having a low non-specific affinity for amide group-containing substances.

The diagnostic devices embodying the present invention provide features which are highly desirable in such test devices. More specifically, the present invention permits rapid, uniform, and reproducible diagnostic tests to be conducted, thus providing a degree of reliability not generally encountered in diagnostic test devices known heretofore. This results from the inclusion of a porous multiple element structure or element which rapidly and uniformly removes the liquid component of a liquid test sample from a reaction zone or that zone where a visible indicia of a positive test may be observed.

A diagnostic device embodying the present invention can include a housing containing a reservoir or receptacle formed therein, such as a tube or well, having a multiple element diagnostic structure located within the housing. The multiple element structure includes a three element embodiment and a two element embodiment. The first or uppermost element of either embodiment of the multiple element diagnostic structure is a microporous, liquophilic membrane, preferably a hydrophilic membrane, which is fixed within the reservoir and defines the bottom of the well or receptacle.

Located below and in contact with the microporous membrane in both embodiments of the present invention is a porous, fibrous, wettable or liquophilic structure typically in the form of a web or mat. In the two element device, this structure serves both as a liquid absorbent element and as a liquid pathway to conduct liquid away from the microporous membrane and toward the bottom of the receptacle or housing in which the two element structure is located. Unlike conventional structures, the inclusion of this liquophilic or wettable element substantially reduces or eliminates the formation of air bubbles at the lower interface of the microporous membrane. In the embodiment which includes a three element diagnostic structure, the preferred embodiment of this invention, the same type of liquophilic, liquid absorbent element is located immediately below and also in contact with the microporous membrane. This liquophilic, liquid absorbent element, for convenience and for reasons which will become immediately apparent, will be referred to as the "first liquophilic, liquid absorbent element" or by a like term, regardless of the embodiment being discussed. In the three element embodiment of the invention, this first liquophilic, liquid absorbent element provides a liquid pathway between the microporous membrane and a second liquid absorbent element, discussed below, located therebelow, for con ducting liquid in a uniform, continuous manner between the reaction zone or retention surface and the second liquid absorbent member of the three element device. The transmission of liquid is provided both by the structure and composition of the intermediate or first porous, liquophilic element. The non-woven fibrous first liquophilic, liquid absorbent structure serves to fill and bridge any voids between the microporous membrane and the second liquid absorbent element. In addition, the fibers of this material extend from the surface of the first liquophilic, liquid absorbent or intermediate element like hairs or tendrils to contact the membrane positioned above the structure and, when present, to engage and intermingle with the fibers of the second liquid absorbent element located below the intermediate or first, liquid absorbent, wettable structure.

As indicated above, located below the first liquophilic (or wettable) liquid absorbent element in the three element embodiment is a second or supplemental liquid absorbent member, typically of a porous nature and formed from nonwoven fibers. This element serves to absorb the bulk of liquid which has passed through the microporous membrane from the liquid test sample initially placed in the well above the membrane.

4

The desirable liquid transmission or conducting properties of the intermediate or first liquophilic, liquid absorbent layer are also believed to result from the chemical composition of such fibers. A surface active or wetting agent is combined with a thermoplastic polymer, typically a liquophobic material. Although not wishing to be held to a particular theory, it is believed that the chemical composition of these fibers assists rapid liquid transfer in several ways. First, the wetting agent which forms a component of the fibers assists ·in thoroughly wetting or rendering the fibers liquophilic. Secondly, the nature and concentration of the particular wetting or surface active agent employed permits a minuscule amount of the surface active agent to be leached from the fibers when contact with a liquid first occurs. The minuscule amount of surface active agent tends to be conducted with the liquid, closely behind the liquid front, to the absorbent element downstream of the intermediate porous liquophilic member. The continuous pathway and uniform liquid flow provided by the intermediate element between the microporous membrane and the absorbent member is also believed to be assisted by the highly absorbent nature and wicking properties of the first, liquophilic, liquid absorbent structure. Because of the attributes of this element of the diagnostic structure of the present invention, such as hairiness, liquophilicity, and liquid absorbency of the non-woven structure, the means sought to provide uniform flow conditions in similar, conventional diagnostic devices, typically compressing the adjacent elements together to thereby achieve intimate contact of the various layers, becomes significantly less critical in the subject invention.

As noted above, the difference between the three element and two element diagnostic structures embodying the present invention is that the lowermost element, the supplemental absorbent member, is deleted in the two element device. That is, the two element embodiment includes a microporous, liquophilic membrane as an upper element and a porous, fibrous liquophilic structure (the first liquophilic, liquid absorbent element) located below and in contact with the microporous membrane. Since the two element structure relies solely on the first liquophilic, liquid absorbent element for liquid absorbency, this element may be slightly thicker in the two element embodiment than in the three element embodiment. However, differences will also depend on the type of samples being examined, the test being conducted, and the volumes of liquid being used.

In each embodiment, the porous multiple element structure is located at the bottom of the receptacle or within a lower portion thereof.

The microporous, liquophilic membrane of the multiple element structure can serve preferably as a reaction zone or layer by incorporating a test reagent on its surface, or as a retention layer to support test reagent-containing particles, such as plastic spheres or beads, for example, polystyrene beads. The membrane also functions as a liquophilic, or wettable, member which permits the liquid of a test sample to flow from the receptacle or well.

Since many of the test samples being analyzed are biological liquids which contain proteinaceous materials that are capable of being nonspecifically bound to the materials used to form diagnostic test devices, it is generally highly desirable to minimize potential interference from such proteinaceous substances which could produce invalid test results. Accordingly, the microporous membrane and other elements of the diagnostic test structure may be treated to reduce non-specific binding, such as by a blocking treatment. Alternatively, materials may be chosen which inherently have a low affinity for amide group-containing materials, such as proteinaceous materials. Even though the diagnostic test reaction is intended to and takes place primarily at the reaction layer of the liquophilic, microporous membrane, or the reagent- containing beads supported by the membrane, interference may result in conventional structures for several reasons. The membranes and the liquid absorbent structures located therebelow are typically formed from materials which become translucent when wet. Thus, even if the liquophilic, and preferably hydrophilic, membrane alone or both the membrane and, when used, the porous, second liquid absorbent structure have undergone a blocking treatment, because of either the color of some of the reagents themselves or reactions occurring below the membrane between proteinaceous materials and test reagents, interfering colors may be viewed through the translucent materials. Because of several properties of the materials used in the first liquophilic, liquid absorbent element of the present invention, discussed below, this does not happen.

The first liquophilic, liquid absorbent element is formed from a material which, although being liquid absorbent and readily wettable, has an inherent low affinity for binding amide group-containing materials, particularly proteinaceous type materials, which binding may be measured by the Immersion Protein Binding Assay, described below. Thus, unlike conventional materials used for the same or similar purposes, the non-woven web (or mat) or medium forming same, i.e., the first liquophilic, liquid absorbent element, requires no blocking to obviate protein absorption. Accordingly, a step which is generally required prior to use of conventional diagnostic membranes or the devices in which they are incorporated is eliminated for the first liquophilic, liquid absorbent element of this invention. Therefore, because of the low affinity for amide group-containing substances, proteinaceous materials do not tend to accumulate in the first liquophilic, liquid absorbent element and react with subsequently added reagents.

In addition, the fibrous, porous, liquophilic (and preferably hydrophilic), liquid absorbent media having a low affinity for amide group-containing substances (and preferably proteinaceous substances) employed as the first liquophilic, liquid absorbent element tends to enhance optical isolation so that colored materials which may be formed or collect below the reaction zone of the membrane are not observed and, therefore, do not interfere with the user's judgment regarding a positive test. These materials tend, because of their fibrous hairy nature, to be fluffy and in the thicknesses obtained in the mat weights suitable for the present invention separate the liquophilic membrane from either the second (supplemental) liquid absorbent pad or the bottom of the well, located below the first liquophilic, liquid absorbent element. Furthermore, the media from which the latter element is formed tend generally to be less translucent (i.e., more opaque) than comparable thicknesses of conventional materials. This nature combined with the ability to select appropriate thicknesses of mat based on a knowledge of the reagents used and the analyte being sought helps to select a thickness which enhances optical isolation (i.e., reduces interfering colors).

Thus, a thickness of the first liquophilic, liquid absorbent element will usually be chosen to satisfy other requirements, such as suitable liquid absorbency and also to achieve sufficient optical isolation (i.e., opacity) for a particular diagnostic test, considering the nature and colors of test samples, reagents, and products which indicate a positive indicia. Typically, the minimum web weight of an uncalendered material which may be employed is 43.06 g/m² (4 gm/ft²) which corresponds to a thickness of about 0.033 cm for a mat of the preferred medium formed from the preferred copolymer of ethylene and 1-octene.

To attain the desirable low affinity for amide group-containing materials, particularly proteinaceous materials, and the liquophilic, preferably hydrophilic, nature of the non-woven, fibrous, porous, liquid absorbent first element, the fibers used are formed from a thermoplastic material which incorporates a surface active agent, such as a surfactant or wetting agent. Unlike many conventional nonwoven materials used for similar purposes in diagnostic devices which require binder to hold the fibers together, the porous structure according to the present invention, in which long fibers are mechanically entangled, is free of binder.

The wettability or liquophilicity of a solid structure, e.g., a membrane, is a function of that structure's critical surface energy and the surface tension of the applied liquid. If the critical surface energy is at least as high as the surface tension of the liquid, the liquid will spontaneously wet the solid structure, which may be termed "liquophilic" with respect to that liquid. For example, a porous membrane having a critical surface energy of 72 dynes/cm or higher will be wetted by water which has a surface tension of 72 dynes/cm and, therefore, tend to freely pass aqueous solutions, i.e., the membrane is "hydrophilic". The capability of a porous structure to be wetted by a liquid can be determined by placing a drop of the liquid on the porous structure. The angle of contact provides a quantitative measure of wetting. A very high angle of contact indicates poor wetting and may be used to characterize a "liquophobic" material, while a zero angle of contact defines complete or perfect (liquophilic) wetting. Materials used in the subject invention as the wettable or liquophilic porous liquid absorbent structures, particularly the first liquophilic, liquid absorbent element, and the liquophilic membrane (the uppermost element) are characterized by being readily or spontaneously wetted by the applied liquid and have a low angle of contact with the applied liquid. Indeed, when a drop of a test liquid(s) is placed on the first wettable or liquophilic, liquid absorbent layer of the porous structure, the drop of liquid penetrates the layer and provides a low angle of contact with the upper surface of the porous structure or membrane.

As used herein, "analyte" refers to any substance or substances being analyzed for or determined by a diagnostic test. The presence of an analyte is typically indicated by the appropriate detection and/or measurement of a chemical or physical property after reaction or complex formation between the analyte and a detection reagent. Examples of properties which may be observed include color changes, radioactivity or enzyme reactions. The terms "reagent", "test reagent", "detection reagent", and like terms refer both to substances reacting directly with an analyte as well as to substances used to convert either another substance or the analyte to a substance suitable for providing an indication, typically an optical indication, of the presence of the analyte. The term includes simple chemical substances, including elements and compounds, of an ionic or molecular nature as well as more complex or macromolecular structures, such as proteinaceous materials, and includes substances of a biological nature such as antigens, antibodies, enzymes, antibody-enzyme conjugates, haptens, receptors, lectins, gene probes, and the like, as well as viruses and bacteria. The analyte may also be one or more of the substances falling within the definition of a reagent. In the case of antibodies, reagents and analytes may each be monoclonal or polyclonal antibodies. Additionally, the analyte may include drugs, peptides, cells, and organelles. A reagent may additionally include buffers, indicator molecules, and substrates.

The term "proteinaceous materials", as used herein, refers to compounds which contain a peptide bond or linkage and includes proteins and polypeptides. The term may also include substances not primarily proteins but w' ich have sterically accessible amide moieties or polypeptide fragments.

Terms such as "surface", "porous structure surface", "media surface" or like terms, used in the singular or plural, are intended herein to include not only the gross surfaces, i.e., the external or outer surfaces, such as those which are exposed to view, but also the internal surfaces or those surfaces which define the pores of the porous, fibrous structure or membrane. Thus, the porous, fibrous structure or membrane surface is that portion of the porous, fibrous structure or membrane which is capable during use of being contacted by a fluid, particularly a liquid. As distinguished from the porous, fibrous structure surface area, which refers to the area of both internal and external surfaces, the exposed planar dimensional area of the material (i.e., the area of the gross surface) is herein referred to as the porous, fibrous structure area or membrane area or like terms.

The terms "web" and "mat", as employed herein in describing the liquid absorbent, porous media employed as the first liquophilic, liquid absorbent element of the present invention, refer to non-woven porous structures in which the structural integrity is attributable to entanglement of fibers. Webs, which are frequently thinner than mats, are often formed from a single layer of fibers. Mats are typically formed from several layers of fibers or webs.

The terms "specific binding", "specific protein binding", and like terms, refer to bonds formed between members, typically proteinaceous members of a biospecific complex involving both molecular forces, other than covalent forces, and the complementary mating of configurationally commensurate portions of molecules. The terms "non-specific binding", "non-specific protein binding", "binding in a non-specific manner" and like terms, refer to bonds formed between a peptide group-containing material, preferably a proteinaceous material, and another substance involving the same type of molecular forces but lacking the configurational interactions found in specific binding.

A diagnostic device embodying the present invention includes what might be described as at least one receptacle or reservoir in which the walls of the receptacle are formed from a liquid impervious material, inert to solvents, reagents, and like materials used in the manufacture of the device or in diagnostic tests for which it is used. Typically an inert plastic, such as polystyrene, polyolefins (polyethylene and polypropylene, for example), or the like, is used. Defining the bottom of the receptacle is a liquid absorbent, porous, multiple element structure. In its simplest form, the diagnostic device may simply be at least one tube having at least one element of the multiple element diagnostic structure, for example, the microporous membrane (the uppermost element), disposed across and secured to one of the open ends of the tube. The other element(s) of the diagnostic structure, the porous, liquophilic, liquid absorbent member (the first liquophilic, liquid absorbent element) and, when present, the second or supplemental absorbent element are located below the membrane, typically within a chamber in the bottom of a housing in which the reservoir is formed. Other alternatives exist, however. For example, the porous two or three member diagnostic absorbent structure may be disposed within the receptacle or tubular structure, at least the microporous membrane being secured to the walls thereof such that the upper and lower gross or external surfaces of the microporous membrane are at substantially right angles to the longitudinal axis of the tube. Such an arrangement permits the porous structure to be located below or recessed from the upstream or inlet opening of the reservoir but also, if desired, above and recessed from the outlet or downstream side of the diagnostic device, thereby permitting amounts of liquid exceeding the capacity of the porous, absorbent structure to be directed to a liquid receiver. Another alternative includes a housing having an exterior and including a chamber within the housing having one portion open to the exterior of the housing and another portion closed from the exterior of the housing and at least a portion of the porous, absorbent, wettable multiple element structure, such as the membrane, disposed across the open portion of the chamber, the uppermost surface of the membrane communicating with the exterior of the housing and lowermost surface of the lowermost element of the two or three element diagnostic structure communicating with the closed portion of the chamber. While the above described structures are preferred for the diagnostic devices embodying the present invention, this invention may be embodied by diagnostic devices having other structures, particularly those in which a well-type arrangement, having a two or three element structure defining the bottom wall of the well, is employed.

Microporous, Liquophilic Membrane:

The uppermost element of the diagnostic structure embodying the present invention is a wettable, i.e., liquophilic, preferably hydrophilic, microporous membrane. Any material is suitable for use as the microporous membrane which does not adversely react with test samples, liquids used in test samples, diagnostic reagents, solvents employed with diagnostic reagents, or other materials which may come in contact with the membrane during the course of preparation or use of the diagnostic device of the present invention. The material should also be capable of supporting plastic reagent-containing beads when such beads are used with the present invention or, while being chemically inert toward the aforementioned substances, may have some selective affinity for the analyte or a test reagent or a reagent used to form a bond with one of the aforementioned to permit immobilization thereon.

Examples of materials which are suitable for use as the wettable, microporous membrane include liquophilic forms of polyamides, polyvinylidene fluoride, and cellulose compounds, such as esters of cellulose, as for example, cellulose acetate, hydroxyalkyl celluloses, nitrocellulose, and mixed esters of cellulose, as well as papers and glass fiber mats with suitable binders and wettable polyethylene. Hydrophilic, microporous polyamide membranes, particularly nylon 66 membranes, are especially preferred.

A preferred microporous, hydrophilic nylon 66 membrane material having high binding capacity, uniformity, controlled pore size, and high surface area is BIODYNE™, available from Pall Corporation and described in U.S. Patent 4,340,479.

Another preferred membrane useful as the indicator medium or reaction layer is IMMUNODYNE™, available from Pall Corporation. IMMUNODYNE™ is a modified CARBOXYDYNE[R] membrane, also available from Pall Corporation. CARBOXYDYNE[R] is a hydrophilic, microporous, skinless nylon 66 membrane with controlled surface properties formed by the cocasting process described in U.S. Patent 4,707,266, as discussed below, specifically by cocasting nylon 66 and a polymer containing an abundance of carboxyl groups to form a membrane having controlled surface properties characterized by carboxyl functional groups at its surfaces. IMMUNODYNE™ membranes are chemically active membranes which may be prepared from CARBOXYDYNE[R] membranes by treating them with a reagent such as trichloro-s-triazine in the manner described in U.S. Patent 4,693,985, discussed below.

Polyvinylidene fluoride membranes are not inherently water-wettable but can be rendered such by an appropriate surface treatment. Microporous, polyvinylidene fluoride membranes which have been treated to render them hydrophilic are commercially available. As discussed above, wettability or liquophilicity is a function of the critical surface energy of the solid structure and the surface tension of the liquid. Wettability may also be expressed in terms of intrusion pressure required for liquid to penetrate into the pores of the membrane. Although a function of the properties of the liquid used, such as surface tension, membrane materials which are particularly preferred have intrusion pressures of, or close to, zero.

Membranes useful as the porous structure in the subject invention typically have large surface areas, e.g., from 2 to 10 $m^2$/gram or even higher. As discussed above, the term "surface area" refers not only to the gross surface(s) of the porous structure but also to the surfaces of the micropores, i.e., the interior surfaces of the structure which are contacted by fluid during use. This characteristic permits a greater amount or higher concentration of reactant to be immobilized in the indicator medium. Accordingly, higher sensitivities may be achieved using the diagnostic device embodying the present invention.

Also included among the preferred polyamide membranes for the present invention are hydrophilic, microporous, skinless polyamide membranes with controlled surface properties of the type described in U.S. Patent 4,707,266 and in U.S. Patent 4,707,840. These hydrophilic, microporous, substantially alcohol-insoluble polyamide membranes with controlled surface properties are formed by cocasting an alcohol-insoluble polyamide resin with a water-soluble, membrane-surface-modifying polymer having functional polar groups. Like the preferred hydrophilic, microporous nylon membranes which do not have controlled surface-modified polar groups present, the polyamide membranes used as the indicator medium or reaction layer in embodiments of the present invention having controlled surface properties are also skinless; that is, they are characterized by through pores extending from surface to surface which are of substantially uniform size and shape. If desired, however, materials having tapered through pores, i.e., pores which are larger at one surface of the sheet, narrowing as they approach the opposite surface of the sheet, may be used.

The surface-modifying polymers used to prepare the polyamide membranes with controlled surface properties, useful in embodiments of the present invention, comprise polymers which contain substantial proportions of chemical functional groups, such as hydroxyl, carboxyl, amine, and imine groups. As a result, the membranes include, at their surfaces, high concentrations of functional groups such as hydroxyl, carboxyl, imine, or a combination of any of the above groups which do not react with one another. These polyamide membranes having controlled surface properties have higher concentrations of carboxyl or imine groups at their surfaces than the preferred microporous, hydrophilic, skinless polyamide membranes described above which do not have controlled surface properties, i.e., those which are formed from the preferred polyamide resin but are not cocast with surface-modifying polymer.

The membranes used may be treated by any method known to one of skill in the art to deposit and/or bind reagents thereto. As indicated above, the reagent may be of an ionic, molecular, or macromolecular nature. When used as a diagnostic tool to provide a visible change, the reagent may be one or a combination of substances which is initially colorless and which, upon reaction with a suitable material, provides an optically measurable response. Other possible variations include the use of suitable labels, such as the formation between the deposited reagent and the material for which testing is being conducted or a complex or compound which is appropriately la beled by any known technique, such as enzymatic/substrate labels or the like.

Although treatment of the porous liquophilic membrane of the diagnostic device with a suitable reagent-(s) may be performed at the time at which diagnostic tests are to be performed, specifically by addition of the test reagent(s) immediately preceding contacting of the test device with the liquid containing the sample to be analyzed for, the present invention may have application to a diagnostic device prepared by treatment of the membrane with the test reagent(s) prior to the manufacture of the individual diagnostic devices.

A useful method of binding reagents of a molecular nature, especially macromolecules, and particularly those of a biological nature, is disclosed in U.S. Patent 4,693,985. This patent describes a method for immobilizing a wide range of biologically active substances as acceptor molecules on active membranes. The acceptor-bound membranes described in the patent are capable of immobilizing and binding a wide variety of biologically-active compounds, specifically ligands, to the acceptor molecules. Using such reaction layers or membranes in the porous structure permits the testing of bodily fluids, such as blood, serum, plasma, urine, saliva, and the like. Use of such membranes also permits testing for particular substances by chemical assays or immunoassays, such as those where a specific label is employed, such as one indicating enzyme activity or an electromagnetic energy absorbing and/or emitting label, such as a fluorescent label. The macromolecules which are used as reagents and bound to the surfaces of the microporous membrane or which are assayed for using the device of the present invention generally include materials of a biological nature and are frequently proteinaceous in nature. The reagent or acceptor molecule bound directly to the reaction layer or the ligand being tested for includes immunoglobulins or antibodies, either polyclonal or monoclonal, antigenic substances, apoproteins, receptors, glycoproteins, lectins, carbohydrates, hormones, enzymes, carrier proteins, heparin, coagulation factors, enzyme substrates, inhibitors, cofactors, nucleic acids, etc.

Porous, Liquophilic, Fibrous Member Having Low Affinity for Protein:

The porous, liquophilic member employed as the first liquophilic, liquid absorbent element (the intermediate structure in the three element structure or the absorbent element in the two element structure) in the diagnostic device embodying the present invention is a non-woven fibrous, preferably microfibrous, member or structure which is liquophilic (i.e., wettable). The structure has a low affinity for amide group-containing, particularly proteinaceous, materials, is also, preferably, hydrophilic and an optical barrier.

The pore rating selected for the medium employed as the first liquophilic, liquid absorbent element will depend on the thermoplastic material used to form the fibers or microfibers and the particular application to which the diagnostic test device will be put. However, pore ratings are typically in the range of 0.5 to 200 microns, preferably 1 to 20 microns. Pore ratings may also be varied across the thickness of the porous structure from one gross surface to the other. This may be achieved by means of a graded or stepped porous, liquid absorbent structure in which multiple webs are superposed on one another to form the composite porous structure. The pore rating of the porous structure may be controlled by calendering. When the same chemical composition is used throughout the stepped pore rating medium, other methods besides the preferred calendering may be used to obtain such media. Thus, variations in belt speed, rate of resin extrusion, resin supply pressure, and diameter of nozzle opening may be used. Although uncalendered porous structures are generally preferred because of higher liquid absorbency, a preferred embodiment of the present invention includes a porous "composite" unitary structure in which a calendered portion of the structure is located at or adjacent one gross surface of the porous medium and the remainder of the structure is uncalendered and inseparable therefrom. Typically, when viewed in cross-section, the calendered portion amounts to 10-35% of the thickness of the web and is generally, preferably, less than 20%. The thickness of the mats of the present invention may vary depending on the application. Typically, the thickness is not more than about 12 mm (1/2 inch).

Absorbency of the porous, fibrous, liquophilic first liquophilic, liquid absorbent element is due primarily to the nature of the absorbing material employed and both the void volume and gross volume, or thickness, of the porous structure. The last two characteristics, void volume and gross volume, also known as "bulk characteristics", can be controlled in the manufacturing process to yield a uniform product which provides reproducible results. Individual webs may be produced having a "web weight" in the range of 5.4 to 107.6 grams/meter$^2$ (0.5 to 10 grams/foot$^2$) of medium and a void volume of 30 to 90%, preferably 50 to 80%. When preparing the porous, first liquid absorbent, liquophilic elements, several passes through the apparatus employed in their preparation creates a multi-layered or multi-web porous structure or mat which is, in most instances, the desirable form of the porous structure. The web weights or, alternatively, "mat weights" will, of course, depend on the thickness of the final porous structure which may be selected based on the application in which the mat is expected to be used. Suitable web weights for the mats typically range from 21.5 to 4300 grams/meter$^2$ (from 2 to 400 grams/foot$^2$) of medium and web weights of 43 to 1076 grams/meter$^2$ (4 grams./foot$^2$ to 100 grams/foot$^2$) are preferred. Suitable void volumes for such layered mats are within the same ranges indicated above for the webs employed in embodiments of the present invention. Manufacturing conditions employed to obtain such mats are discussed below.

As indicated above, the fibrous, and preferably microfibrous, media employed in embodiments of the present invention are liquid absorbent and quite wettable. The latter property is reflected by the Critical Wetting Surface Tension (CWST), which is the surface tension of a liquid that is just capable of wetting a porous structure. The fibrous, liquophilic, porous absorbent media can be prepared to provide suitable wettability for any liquid employed. However, since aqueous solutions are used most frequently in diagnostic tests, the porous structures exhibit CWST values suitably of at least 70 to 72 dynes/cm. Preferably, the porous structures have CWST values of at least 100 dynes/cm.

The absorbent fibrous, liquophilic media which serve as the material for the first liquophilic, liquid absorbent element in embodiments of the present invention also exhibit a low affinity for binding amide group-containing materials, particularly proteinaceous substances in a non-specific manner as measured by the Immersion Protein Binding Assay (IPBA), described below. When tested by this procedure, media which sorb no more than about 85 micrograms of amide group-containing material per cubic centimeter of media are considered to have a low affinity for amide group-containing materials, such as proteinaceous materials. Preferred are very low affinity media, such as those media which bind no more than about 50 micrograms of amide group-containing material per cubic centimeter of media. The media most preferred include those which bind below 30 micrograms of amide group-containing material per cubic centimeter of media. In this procedure, the affinity for amide group-containing materials is indicated by a test wherein protein solutions are applied to a sample of medium, nonbound excess protein is flushed away, and the sample of medium is assayed for bound residual protein.

The IPBA, described in greater detail below, is a radioimmunoassay. To conduct this test, uniform size (for example, 13 mm diameter round discs) samples are cut, identified, and placed in a suitable container along with a solution containing a known amount of unlabelled goat IgG and $^{125}$I-goat IgG in a buffered solution, such as phosphate buffered saline solution (PBS). The solution is counted with a gamma counter prior to contacting the membranes with the solution to determine the ratio of counts to unit of mass of the goat IgG. All of the samples are then placed in the PBS solution containing the labelled proteinaceous material and agitated for a fixed period of time, on the order of about one hour. The solution is then decanted from the samples and the remaining liquid removed by pipet. The media samples are then washed in a PBS solution of the same volume as the goat IgG solution, the samples being agitated in the solution for approximately 5 minutes. The washing procedure is repeated twice more and the samples are removed from the wash solution, blotted, and counted with a gamma ray counter. The amount of protein bound by the membrane is then determined.

In addition to the weight per square foot and void volume, which can be controlled by calendering of the assembled fibrous mat, another important factor in determining pore characteristics is fiber diameter, discussed below.

Thermoplastic Fibers:

Many of the characteristics associated with the porous, liquophilic, liquid absorbent media of the first liquophilic, liquid absorbent element in embodiments of the present invention are attributable to the fibers employed to make these media. These fibers are formed in major part from thermoplastic polymers and exhibit a low affinity for amide group-containing substances, particularly toward proteinaceous materials. Typically, the thermoplastic polymers used to form the fibers of the porous, liquid absorbent media are liquophobic and, in particular, hydrophobic.

Materials suitable as the thermoplastic polymer used in embodiments of the present invention are those materials which are generally inert toward the solvents, reagents, and other substances typically present either in analyte-containing samples or test reagent solutions used in particular diagnostic tests. Preferred for use in embodiments of the present invention are polyolefins, polyamides, polyesters, and copolymers of the aforementioned. Most preferred are polyolefins, particularly polyethylene and polypropylene and copolymers of ethylene or propylene, and other olefins. Such other olefins are preferably $\alpha,\beta$-ethylenically unsaturated alkenes, more preferably those olefins having from 3 to 12 carbon atoms per molecule and most preferably ones with 4 to 8 carbon atoms per molecule. Exemplary of such other olefins is 1-octene. Preferred among the polyolefins and copolymers thereof are linear low density polymers, preferably linear low density polyethylene copolymers (LLDPE, described in U.S. Patent 4,578,414 to Sawyer et al.) obtained by copolymerizing ethylene with other $\alpha$-olefins.

As indicated above, the thermoplastic materials used in preparing the liquophilic fibers for embodiments of the present invention are in most instances liquophobic and typically hydrophobic. This is the case for those materials which are particularly preferred, the polyolefins. Such polymers, while having certain characteristics which make them useful in embodiments of the present invention, because of their liquophobic or hydrophobic nature, are not well adapted, when in web or mat assemblies, to readily pass liquids, such as the type found in test samples. The fibers and porous media formed therefrom must be wettable by the liquids which form the sample solutions or reagent solutions employed in diagnostic tests. Therefore, the CWST of the fibers must equal or exceed the critical surface tension of the liquid employed. In embodiments of the present invention, this is achieved by combining a wetting or surface active agent with the thermoplastic polymer used to form the fibers.

The wettable fibers or filaments employed in embodiments of the present invention to form the liquid absorbent, porous media and structures of the first liquophilic, liquid absorbent element are prepared by compounding a thermoplastic polymer with at least one, and preferably only one, surface active agent. The term "surface active agent" is intended to include wetting agents and surfactants generally. However, the term is not meant to suggest that the surface active agent is formed only as a coating on the surface of the fibers. Rather, because of the way in which the thermoplastic polymer and surfactant are blended and the fibers are formed, some of the surfactant is believed to be contained within the fibers. The preferred surface active agent is a mono-, di-, or tri-glyceride or a mixed glyceride and is preferably a mono-glyceride of a carboxylic acid, particularly a fatty acid. The acids employed may be saturated or unsaturated, preferably the latter. They have a length of about 12 to 22 carbon atoms, preferably about 14 to 18 carbon atoms. The acid most preferred in embodiments of the present invention is Z-9-octadecenoic acid and the surfactant most preferred in embodiments of the present invention is the mono-ester of Z-9-octadecenoic acid and 1,2,3-propanetriol (glycerol).

The surface active agent, or a mixture of such agents, is present in an amount of 0.1 to 5%, preferably 0.1 to 3% by weight, based on the total weight of the polymer formulation. When a polymer formulation which includes a polyolefin is used, particularly one which employs polyethylene or a copolymer of ethylene and the preferred $\alpha,\beta$-ethylenically unsaturated alkene, it is preferable to use 0.25 to 2%, by weight, of the surface active agent. Although higher amounts of the surface active agent may be used, it does not appear beneficial to do so and a greater expense is incurred. In addition, using higher amounts of the surface active wetting agent runs the risk of incurring more than the minimum acceptable extraction of surface active agent during a treatment or test procedure. Most preferably, about 1% of the surface active agent is employed when formulating the LLDPE polymer formulations used in preparing the fibers of the second element.

In formulating the compositions used to prepare the fibers of the first liquophilic, liquid absorbent element, it is preferred to use concentrated master batches of blends of polymer and surface active agent which are subsequently blended, as portions, with additional quantities of barefoot (uncombined) polymer resin. This results in a polymer formulation which incorporates the surface active agent in a more dilute form directly in the bulk polymer resin. Alternatively, the surface active agent may be mixed in pure form with the polymer while the latter is in a molten state. The various additives also may be added sequentially.

A preferred polymer formulation useful in preparing the fibers which form the nonwoven porous structure comprising the first liquophilic, liquid absorbent element includes a copolymer formed, by weight, from a major portion (above about 90% and preferably greater than about 94%) ethylene and a minor amount (less than about 10% and preferably less than about 6%) of octene-1. Typically, this copolymer, containing no surface active agent, is mixed with a portion of copolymer containing about 5% of the preferred surface active agent, 1,2,3-propanetriol mono ester of Z-9-octadecenoic acid. The proportions of copolymer containing surface active agent and "pure" copolymer are mixed to provide the desired concentration of surface active agent.

The polymer formulation may be prepared by any convenient method typically used in compounding thermoplastic polymer formulations. The method suggested in U.S. Patent 4,578,414 may be used to advantage. Specifically, the surface active agent may be combined with the polymeric material when in a molten form by mixing the materials according to commonly used techniques and equipment. Examples of such techniques include roll-milling, mixing in a Banbury type mixer, or mixing in an extruder barrel, etc.

The porous, liquophilic absorbent webs of the first liquophilic, liquid absorbent element are prepared in a melt-blowing procedure employing a melt-blowing apparatus. This process was first developed by the Naval Research Laboratory and is described in "Manufacture of Superfine Organic Fibers", U.S. Department of Commerce, Publication PB111437 (1954) and in Van A. Wente, "Superfine Thermoplastic Fibers" (August 1956). In such process, the polymeric material or, more preferably, the polymeric resin which includes the blended wetting agent is heated until molten and extruded through multiple nozzles under pressure. A stream of hot air is directed toward the emerging molten material to entrain the emerging fine fibers. Control of fiber size is achieved as a result of manipulating the resin pressure, the air pressure or velocity, and the temperature. The conditions are varied to obtain microfiber diameters preferable in embodiments of the present invention. Typically the fiber diameters are on the order of 0.5 to 20 microns, preferably 1 to 8 microns.

The apparatus is adjusted so that the microfibers are directed to impact on a collection surface to form a web. As the fibers strike the collection surface they become mechanically intertwined with one another, forming a coherent mass of microfibers of great uniformity. The conditions are adjusted so that little if any fusion occurs between the individual microfibers, since excessive fusion results in webs which are undesirably stiff and brittle and create problems in manufacture and later handling of the web.

Typically, the collection surface is a moving belt formed from a material which exhibits little adhesion to the microfibers that impact on its surface. Alternatively, a substrate for the microfibers may be employed which permits the microfibers to intertwine with the substrate, producing a web which has a greater strength than the microfibers alone. Such substrates may be composed of substances which, like the thermoplastic resins combined with the surface active agents, are chemically inert to the solvents, test reagents, or components found in either the analyte, test solutions, or solvents employed in the manufacturing or testing processes. Examples of suitable substrates include polyesters and polyolefins, and may be in the form of a woven or nonwoven matrix. The substrate may also be another web or mat of the non-woven, porous, absorbent mediumon embodiments of the present invention formed of the same or different material. Typically the material is chemically the same but the pore rating is different. The latter characteristic is generally achieved by calendering. Thus, a stepped or graded pore rating may be provided in a composite medium.

The belt speed, in combination with the rate of extrusion of the resin and the velocity of the airstream, determines the web weight per square foot. Web weights in the range of 1.08 to 108 g/m$^2$ (0.1 to 10 grams/foot$^2$) of medium are typically obtained in a first pass through the apparatus when the first layer is obtained. Multiple passes are preferred to both increase the web weight and enhance uniformity of the resultant mat. Most preferred are mats in which the fibers impact on the previously formed web or layer with the opposite orientation (left for right or 180° from the first orientation) during subsequent passes. With repeated passes, virtually any desired web weight can be achieved for the finished product. In embodiments of the present invention, web weights of the mats of 21.5 to 4306 g/m$^2$ (2 to 400 grams/foot$^2$) of medium are preferred and most preferred are web weights of 43.1 to 1076 g/m$^2$ (4 to 100 grams/foot$^2$).

In some cases, it may be desirable to provide a graded surface wherein the upper surface of the web or mat is coarser, having larger pores, and the lower layers are finer, having smaller pores. This structure may be used in some situations where restricted or slower flow through the reaction membrane is desirable. This permits enhanced sensitivity in those tests which require longer reaction times for reactions between the test reagent and analyte. Such embodiments can be achieved by variation in the resin supply pressure, the nozzles employed and/or the air pressure during web formation. This results in variations in the diameter of the fibers. Alternatively, a calendering process may be employed to form the stepped or graded medium.

In altering the pore rating and void volume by compression of the porous, absorbent structure of the first liquophilic, liquid absorbent element, the most convenient method is a calendering process employing heat and pressure in which the porous structure is passed between heated rolls. Preferred in embodiments of the present invention is an apparatus in which one roll is steel and the other is compressed cotton. A temperature is used which is just below the softening point of the thermoplastic polymeric material. The objective is to employ a combination of temperature and pressure which will result in a permanent deformation of the fibers but will not produce significant fusion and fiber-to-fiber bonding, thereby resulting in a mat in which the integrity is determined by mechanically intertwined fiber rather than bonded or fused fibers.

The present invention may be employed to produce uniform, porous, absorbent webs which may be used in diagnostic devices to produce uniform and reproducible test results. Such uniformity in the mats produced and the concomitant test results is determined in part by the ability to control fiber diameters and the production of uniform fiber diameters in any or all layers of the medium. The uniform nature and performance of mats also results from the production of consistent web weights, that is, mats having reproducible and uniform densities as determined by taking a cross-sectional sample from any part of a roll of media which may be. compared to a sample taken from another part of the roll. This is achieved by multiple laydowns or passes through the apparatus in which the fibers are extruded.

Supplemental Liquophilic, Liquid Absorbent Element:

In many instances, when the volumes of liquid associated with samples being tested and any reagents used are small, the two element diagnostic test structure of the present invention is quite adequate to absorb all of the liquid. Even in those situations where larger volumes of liquid are employed, due to the nature of the first liquophilic, liquid absorbent element, and selection of an appropriate thickness of this element, the two element embodiment of the diagnostic device may be effective in removing all liquid from the test or reaction zone of the porous, liquophilic membrane. However, in many instances, particularly those in which large volumes of liquids are used, a supplemental or second liquophilic, liquid absorbent element, placed below and in contact with the first liquophilic, liquid absorbent element may be desirable.

Suitable for use as this second element is any material which is fibrous, and preferably in the form of a non-woven web, which is also liquophilic, preferably hydrophilic, and liquid absorbent. The material should also be one that does not adversely react with test samples, liquids used in test samples, diagnostic reagents, solvents employed with diagnostic reagents, or other materials which may come in contact with the element during the course of preparation or use of a diagnostic device embodying the present invention. Examples of materials which are suitable for use as the second or supplemental liquophilic, liquid absorbent element are wettable forms of cellulose compounds, such as esters of cellulose, as for example, cellulose acetate, hydroxyalkyl celluloses, and mixed esters of cellulose, as well as papers with suitable binders. Most preferred is cellulose acetate. The aforementioned materials are preferably used as mats, wads, or plugs.

Example 1: Preparation of a Wettable Polypropylene Mat

A wettable polypropylene was prepared containing 99.5%, by weight, of Exxon polypropylene (grade 3045) and 0.5%, by weight, of Atmer. 645 wetting agent. The latter is a complex mixed glyceride with a long chain (12 to 16 carbon atoms) fatty acid adduct, available from ICI America Inc. The mixture was prepared by blending 18.14 kg. (40 pounds) of Exxon polypropylene, with 0.11 kg. (0.25 pounds) of Atmer 645 wetting agent. This mixture was tumbled in a rotating drum mixer for 30 minutes to produce pellets, which contained the wetting agent on the surface thereof.

These pellets were heated to 343.3° C (650° F), and fiberized by passage through a melt blowing die. The resin pressure was 140,620 kg/square meter (200 psi) and the air pressure 15,468 kg/square meter (22 psi). The emerging fibers were collected on a polypropylene non-woven substrate (Lutrasil 5020). Fibrous webs with weights of 21.5, 43.1, and 86.1 gram/meter$^2$ (2, 4 and 8 grams/foot$^2$) were collected. The non-woven substrate was stripped from the web prior to tests and use.

The CWST of these webs were measured and found to be 72 dynes/cm. The wetting point for water requires a CWST of 72 dynes/cm, which means that the webs were just water-wettable. A web prepared from Exxon polypropylene alone measured 28 to 31 dynes/cm, and thus would not wet with water.

Example 2: Preparation of a Wettable Mat of Polyethylene and Octene-1

A wettable polyethylene was prepared from a co polymer of, by weight, 94% ethylene and 6% octene-1 (the copolymer is available commercially from Dow Chemical Company as Aspun 6809). To this copolymer was added 1%, by weight, of a monoester of Z-9-octadecenoic acid and 1,2,3-propanetriol as a wetting agent (commercially available as XU 61518.10 from Dow Chemical Company).

This mixture was prepared by a commercial compounder who blended 80 parts of Aspun 6809 with 20 parts of a stock concentrate, XU 61518.10, the total weight being 226.8 kg. (500 pounds). (The stock concentrate is itself a mixture of 95% Aspun 6809 and 5% of the monoester of Z-9-octadecenoic acid with 1,2,3-propanetriol.) Thus the final composition of the mixture was 99% Aspun 6809 with 1% wetting agent.

Mixing was accomplished by first tumbling the components in a Banbury Mixer for 30 minutes. The mixture was then turned into a homogeneous blend by passage through an extruder at a temperature just above the melting point of the thermoplastic polymer, at about 205°C (400°F). (This low temperature is preferred to minimize degradation.) The extrudate was then cooled and chopped into pellets, rebagged and returned.

The returned pellets were then heated to 321°C (610°F), and fiberized by passing through a melt-blowing die at 105,465 kgs/square meter (150 psi) resin pressure and 14,062 kgs/square meter (20 psi) air pressure. The emerging fibers were collected on a non-woven polypropylene substrate (Lutrasil 5020). In this manner webs of 21.5, 43.1, and 86.1 grams/meter$^2$ (2, 4, and 8 grams/foot$^2$) were prepared. Prior to test and use, the substrate was stripped from the webs.

Measurements were made on webs prepared from the copolymer of ethylene and octene-1 alone (method as above omitting the addition of the wetting agent), and yielded a CWST of 35 dynes/cm. The copolymer with wetting agent, described above, resulted in mats with a CWST of 104 to 115 dynes/cm. Water absorption in these mats was virtually instantaneous.

Example 3: Calendering of Hydrophilic Medium

The melt blown webs were calendered by passing the web through a pair of heated rolls. One of the rolls is highly polished steel, and is heated by a hot oil circulation system. The second roll, in intimate contact with the first, is provided with a compressed cotton outer surface. Temperature and pressure regulating systems allow a wide range of heat and pressure to be applied to the pair of rolls.

The polyethylene web described in Example 2 was calendered at a roll temperature of 93°C and a pressure of 20 tons. The web speed was 1.49 meters/minute (16 feet/minute) through the nip, or intersection, of the calendering rolls. When tested for water bubble point (a measure related to the pore size of the web), the 86.1 gram/meter$^2$ (8 gram/square foot) sample went from a pre-calendered 330.2-381 mm. (13-15 inches) water bubble point to approximately 1016 mm. (40 inches) water bubble point. This indicated that the calendering had considerably decreased the pore size.

Example 4: Measurement of Protein Binding

The microporous, hydrophilic structures prepared according to Examples 2 and 3 as well as known nylon membranes were tested for their abilities to bind the protein goat immunoglobulin G (goat IgG) according to the general procedure for measuring protein binding outlined below.

Immersion Protein Binding Assay

To determine the affinity of microporous wettable webs for protein containing materials, a radioimmunoassay was performed on several samples of webs prepared in Examples 2 and 3. In addition, the same procedure was used to determine the affinity of nylon membranes for protein containing materials. Included in the nylon membrane group were nylon membranes chemically activated (modified to enhance protein attachment) and those having surfaces modified to minimize affinity for or binding of protein.

To perform the test, 13 mm diameter disks were punched from each sample using a cork borer. For identification, nylon disks were labelled by marking with a pencil and disks of hydrophilic polyethylene webs were notched with a scissor. Two disks per sample were used.

A solution was prepared by combining 10 μg/ml of goat IgG (Sigma Chemical Company) and a solution of $^{125}$I-goat IgG (New England Nuclear Corp.) in phosphate buffered saline solution (PBS). This mixture was counted in a gamma counter and yielded a count of 50681 counts/minute/ml. Dividing by the mixture concentration of 10 μg/ml gives 5068 counts/minute/μg.

The biological protein material in PBS was adjusted to a pH of 7.0 with a solution containing 0.15 moles/liter sodium chloride and 0.02 moles/liter of a mixture of mono-, di-, and trisodium phosphate. In those situations in which more than one sample of media was tested at the same time, all samples of media were immersed in the same portion of solution and the volume of the total solution adjusted to provide 2 ml of solution per 13 mm disc.

The discs were agitated in the solution for a period of one hour, after which the IgG solution was decanted and the remaining liquid was removed from the immersion vessel using a pipet. PBS solution was added to the immersion vessel in the same volume as the previously used biological material-containing solution (2 ml per disc) and the membranes were agitated in the solution for 5 minutes to wash and remove residual protein. The wash solution was decanted and the washing procedure was repeated twice using fresh PBS solution each time for a total of three washes. The membrane discs were then removed, blotted gently between absorbent paper, and the residual activitiy was counted using an LKB Wallac Mini Gamma Ray Counter, model no. 1275. The amount of radioactive material on the disc reflects the total protein bound to the discs, i.e., both covalently bound protein in those discs which were chemically active and that which is present because of strong non-specific absorption.

Protein bound/unit volume of membrane was calculated by dividing the counts obtained after the PBS wash by 5068 counts/minute/$\mu$g, then dividing by the volume of the individual 13 mm diameter disks. Volume is calculated by the formula: ($\pi$ x $d^2$)/4 x thickness of the sample.

The data collected and calculated as described above are as follows:

Table 1

| Sample | Mat Weight (g/m²) | Thickness (cm) | Counts | Protein Bound/unit volume of Membrane ($\mu$g/cm³) |
|---|---|---|---|---|
| A | 43.1 | 0.015 [a] | 2510 | 25 |
| B | 86.1 | 0.025 [a] | 3909 | 23 |
| C | 43.1 | 0.033 | 4400 | 20 |
| D | 86.1 | 0.064 | 9326 | 22 |
| Immunodyne™ [b] | | 0.0165 | 29607 | 267 |
| Biodyne™ A [c] | | 0.0165 | 34585 | 312 |
| LoProdyne™ [d] | | 0.0165 | 9443 | 85 |
| Blocked[e] Immunodyne™ | | 0.0165 | 2822 | 25 |
| Blocked[e] Biodyne™ A | | 0.0165 | 2903 | 26 |
| Blocked[e] LoProdyne™ | | 0.0165 | 2545 | 23 |

[a] calendered

[b] Immunodyne™ membranes are nylon membranes (available from Pall Corporation) surface modified to be chemically active for formation of covalent bonds with proteinaceous materials.

[c] Biodyne™ A (available from Pall Corporation) membranes are nylon 66 membranes which bind protein non-specifically.

[d] LoProdyne™ (available from Pall Corporation) membranes are surface modified membranes modified to minimize the binding of proteins.

[e] The membranes which are indicated as "blocked" have been chemically treated with a 0.5% casein solution prior to contact with the Goat IgG solution to inactivate the structures to protein absorption

Example 5: Determination of Absorbent Capacity (Absorbency) of Polyethylene Webs

In this example, two rectangular pieces were cut from each of five samples: A, B, C, D (all polyethylene melt blown webs), and a sample of Biodyne™ A nylon membrane. For all samples, the length and width were measured, along with the thickness. For greatest accuracy, the melt blown webs were measured by noting the minimum setting on a micrometer that allowed the web to be drawn through the jaws of the micrometer without dragging. For all samples dry weights were determined.

To determine the volume of water that could be held per cubic centimeter of web volume, the following calculation was performed: The volume the fibers alone occupy is subtracted from the volume of the web (membrane) sample (length x width x thickness). The volume of the fibers alone is the dry web weight divided by the density of the polyethylene fibers ($0.93$ gms/cm$^3$ for Aspun 6809). The difference between the volume of the web sample and the volume of the fibers alone equals the void volume of the sample material. Since these fibers are highly wettable, absorbing water immediately upon contact therewith, the void volume of the medium is essentially equal to the retained water volume. The retained water volume divided by the volume of the web sample equals the amount of water that can be retained per cubic centimeter of medium. The calculation for the above sample A of wettable polyethylene indicates a sample void volume which equals the retained water volume of $0.728$ cm$^3$.

The Absorbency is the ratio of the retained water volume divided by the volume of the membrane. For a $1.082$ cm$^3$ sample of the above-described polyethylene sample, an Absorbency was determined to be $0.673$ cm$^3$ water/cm$^3$ web. For comparison, a similar determination was done for a $0.45$ micron pore rated supported Biodyne$^{TM}$ A nylon 66 membrane. For a $1.236$ cm$^3$ sample having a density of $1.10$ gms/cm$^3$ and a polyester support having a density of $1.39$ gms/cm$^3$, an average density of $1.24$ gm/cm$^3$ was used. The sample void volume was $0.792$ cm$^3$ and the Absorbency of the hydrophilic material was determined to be $0.64$ cm$^3$ water/cm$^3$ membrane. The data derived from these determinations are listed below:

Table 2

| Sample | Mat Weight (g/m$^2$) | Retained Water Volume/Volume of Medium (cm$^3$ water/cm$^3$ medium) |
|---|---|---|
| A Calendered | 43.1 | 0.67 |
| B Calendered | 86.1 | 0.63 |
| C Uncalendered | 43.1 | 0.85 |
| D Uncalendered | 86.1 | 0.85 |
| Biodyne$^{TM}$ A | | 0.64 |

The term "uncalendered" as used in these examples refers to the material as laid down in the melt-blowing apparatus. Material indicated as "calendered" includes samples obtained by compressing webs taken from the same rolls as Samples A and B. Calendering was performed as described in Example 3. The Biodyne$^{TM}$ A nylon membrane is included for reference purposes.

As may be noted in Table 2, calendering of the material of Samples C and D to produce the material of Samples A and B results in a decrease in Absorbency while decreasing the pore rating. An Absorbency suitable for many applications of the present invention includes values greater than about $0.6$ cm$^3$ water/cm$^3$ medium. Preferred, however, are values of at least about $0.8$ cm$^3$ water/cm$^3$ medium.

## Claims

1. A diagnostic device comprising:
   (a) a receptacle having an open bottom; and
   (b) a multiple element diagnostic structure secured to said receptacle and disposed across the open bottom, said multiple element diagnostic structure comprising:
   (i) a porous, liquophilic membrane, and
   (ii) a liquid absorbent, wettable, porous element having a low non-specific affinity for amide group-containing substances, as measured by the Immersion Protein Binding Assay located below and in contact with said porous, liquophilic membrane.

2. A diagnostic device according to claim 1 wherein said liquid absorbent, wettable, porous element comprises a non-woven element of thermoplastic, liquophilic fibers having a low affinity for amide group-containing substances.

3. A diagnostic device according to claim 2 wherein said fibers are formed from a surface active agent combined with a thermoplastic polyolefin, polyamide, polyester, or a copolymer derived from monomers used to form the aforementioned.

4. A diagnostic device according to claim 2 wherein said fibers are formed from a surface active agent combined with a thermoplastic polymer of polyethylene or polypropylene or copolymers of ethylene or

propylene and an $\alpha,\beta$-ethylenically unsaturated alkene having from 3 to 12 carbon atoms per molecule.

5. A diagnostic device according to claim 4 wherein said polyolefin is a copolymer of ethylene and 1-octene.

6. A diagnostic device according to claim 2 wherein said fibers are microfibers having an average diameter in the range of 0.5 to 20 microns and said structure is microporous.

7. A diagnostic device according to claim 3 wherein said surface active agent is a mono-, di-, or triglyceride of a carboxylic acid.

8. A diagnostic device according to claim 7 wherein said carboxylic acid is a fatty acid having 12 to 22 carbon atoms.

9. A diagnostic device according to claim 8 wherein said surface active agent is a monoester of Z-9-octadecenoic acid and 1,2,3-propanetriol.

10. A diagnostic device according to claim 1 wherein said multiple element diagnostic structure includes a supplemental porous, liquophilic, liquid absorbent element located below and in contact with said liquid absorbent, wettable, porous element having a low non-specific affinity for amide group-containing substances.

11. A diagnostic device according to claim 1 wherein said liquid absorbent, wettable, porous element has a non-specific binding of amide group-containing substances measured by the Immersion Protein Binding Assay of no more than about 85 $\mu$g of amide group-containing substance/cm$^3$ of porous structure.

12. A diagnostic device according to claim 1 wherein said liquid absorbent, wettable, porous structure has a non-specific binding of amide group-containing substances measured by the Immersion Protein Binding Assay of no more than about 30 $\mu$g of amide group-containing substance/cm$^3$ of porous structure.

13. A diagnostic device according to claim 1 wherein the CWST of said wettable, porous, liquid absorbent element is at least 100 dynes/cm.

14. A diagnostic device according to claim 1 wherein said wettable, porous, liquid absorbent element has an Absorbency of at least about 0.60 cm$^3$ of water/cm$^3$ of media.

15. A diagnostic device according to claim 1 wherein said wettable, porous, liquid absorbent element has an Absorbency of at least about 0.80 cm$^3$ of water/cm$^3$ of media.

16. A diagnostic device according to claim 1 wherein said liquid absorbent, wettable, porous element has pore dimensions varying stepwise between a first surface and a second parallel surface.

17. A diagnostic device according to claim 16 wherein the liquid absorbent, wettable, porous element includes a calendered portion at said first surface and an uncalendered portion at said second surface.

18. A diagnostic device according to Claim 1 wherein said diagnostic device is adapted to be used in conjunction with a test reagent.

19. A diagnostic device according to claim 1 wherein said liquid absorbent, wettable, porous element is located between said porous, liquophilic membrane and a supplemental, porous, liquophilic, liquid absorbent, element and comprises a non-woven microporous structure of thermoplastic, hydrophilic fibers formed from a copolymer of polyethylene and 1-octene combined with a monoester of Z-9-octadecenoic acid and 1,2,3-propanetriol and having an average diameter in the range of 0.5 to 20 microns, said liquid absorbent, wettable, porous element also has a non-specific binding of amide group-containing substances of no more than about 30 $\mu$g of amide group-containing substance/cm$^3$ of porous structure, a CWST of at least 100 dynes/cm, and an Absorbency of at least a major portion of said porous structure of at least 0.80 cm$^3$ of water/cm$^3$ of medium.

20. A multiple element diagnostic structure comprising:

(a) a porous, liquophilic membrane, and

(b) a liquid absorbent, wettable, porous element having a low non-specific affinity for amide group-containing substances, as measured by the Immersion Protein Binding Assay located below and in contact with said porous, liquophilic membrane.

21. A multiple element diagnostic structure according to claim 20 further including a supplemental, liquophilic, liquid absorbent element located below and in contact with said liquid absorbent, wettable, porous element having a low non-specific affinity for amide group-containing substances.